# EUROPEAN PATENT SPECIFICATION

(11) **EP 0 764 034 B1**
(45) Date of publication and mention of the grant of the patent: **29.07.1998**
(21) Application number: 94915494.2
(22) Date of filing: 16.05.1994
(51) Int. Cl.: A61M 5/28, A61M 5/315, A61J 1/06

(54) **IMPROVEMENTS IN PHARMACEUTICAL CONTAINERS**
VERBESSERUNGEN AN PHARMAZEUTISCHEN BEHÄLTERN
RECIPIENTS AMELIORES POUR PRODUITS PHARMACEUTIQUES

(30) Priority: 17.05.1993 GB 9310084
(43) Date of publication of application: 26.03.1997
(73) Proprietor: REYNOLDS, David L., Lac Brome, Quebec J0E 1V0 (CA)
(72) Inventor: REYNOLDS, David L., Lac Brome, Quebec J0E 1V0 (CA)
(74) Representative: Newby, Martin John
(86) International application number: CA9400271
(87) International publication number: WO9426332

(56) References cited:
- EP-A- 0 107 874
- EP-A- 0 511 402
- WO-A-92/08507
- US-A- 5 207 646

## Description

This invention relates to pharmaceutical containers, more specifically bottomless pharmaceutical vials of the general character described in European Patent Application No. 0298585, and International Patent Application WO 92/08507. Such vials once filled and capped can be converted into prefilled syringes forming the basis of a wide range of pharmaceutical delivery systems.

There is an increasing requirement for terminal sterilization of such bottomless vials and prefilled syringes, and even if the sterilization procedure is very carefully controlled using sophisticated equipment, there is a risk of pressure differentials being set up across the piston of the vial or syringe which may result in its expulsion. Such pressure differentials could also conceivably occur during storage, particularly in the case of vials containing large gas spaces or substances which may generate gases during storage.

It has previously been proposed to provide piston retainer rings which interlock projections formed at or near the base of the vial, and which may also serve to provide a finger grip for a syringe formed from such a vial. It is however important that such projections do not interfere to any significant extent with either the stable passage of vials through vial filling and capping machinery, nor with insertion of the piston into the vial body. They must also permit easy insertion of retainer rings and provide for their positive retention once inserted.

Such an arrangement is described in WO 92/08507. A problem with this arrangement is that installation of the retainer rings must be effected between filling and capping of the vials and their terminal sterilization, which introduces a further manufacturing step that must be carried out under clean room conditions.

I have now found that, by further development of the arrangement of WO 92/08507, it is possible to provide a retainer ring which can be inserted prior to filling and capping of the vial without interfering with passage of the vial through the machinery carrying out these operations. This means that prepared presterilized vials with both piston and retainer in place may be utilized in the filling and capping operation to provide an assembly ready for terminal sterilization without any additional operation or any requirement for retainer inserting machinery as part of the filling line.

EP-A-511402 discloses a syringe having a combination finger grip and piston retainer assembly. A two part finger grip having flanges extending beyond the outer diameter of the syringe body is assembled around the rear of the syringe and locked in place by a piston retainer which also extends beyond the outer diameter of the syringe body so as to lock the finger grip in place. The retainer is solely supported by the finger grip and therefore cannot operate in the absence of the finger grip. The arrangement is also dependent upon the presence of an externally extending flange on the syringe body.

EP-A-107874 discloses a plastic ring fitted into the bottom opening of a cartridge as part of an arrangement for reinforcing the cartridge against breakage in an automatic injection device.

The present invention relates to an improved bottomless vial as set forth in the appended claims.

Further features of the invention will be apparent from the following description of reference to the accompanying drawing, wherein:
Figure 1 is a longitudinal section through the body of an embodiment of a bottomless vial, shown fitted with a piston retainer ring; and
Figure 2 is a detail of the body shown in Figure 1, on an enlarged scale, and with an optional finger flange portion shown separated.

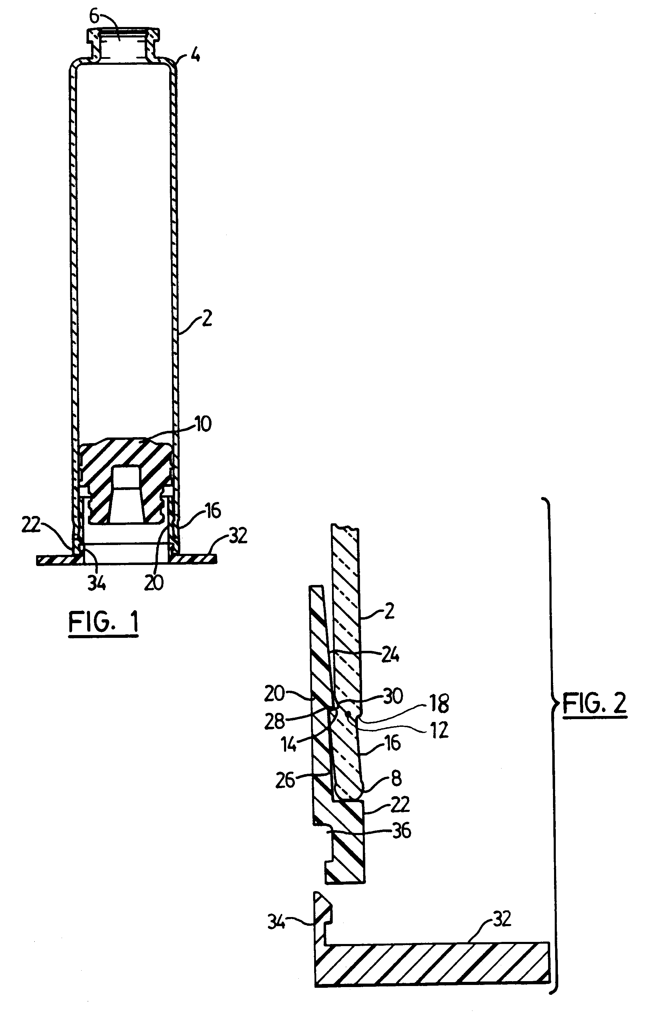

Referring to Figures 1 and 2, a body 2 for a bottomless vial is moulded from glass or synthetic plastic material, with a generally cylindrical form having shoulders 4, at the top end connecting to a hollow neck 6. At a bottom end of the body its side wall is formed with a rounded or beaded bottom edge 8 to form an open bottom end through which a piston 10 may be inserted.

A portion 16 of the inside side wall adjacent the bottom edge 8 is tapered inwardly and joined to the remainder of the side wall by a peripheral joggle 12 so as to provide a narrow internal upwardly facing annular shelf or shoulder 14 above a funnel-shaped upwardly tapered bottom entry to the interior of the body. The shelf and tapered bottom entry may be formed by rolling a heat softened bottom portion of the wall against a suitably shaped mandrel, in which case the outer face of the wall will be recessed as shown at 18, or may be moulded in which case the outer recess may not be present. In either case, there will be no projection, or at least no significant projection, of the outer face of the moulded or rolled portion of the wall outside the circumference of the remainder of the wall. The tapered bottom entry assists in insertion of the piston 10, and subsequent insertion of the retainer ring 20 described below.

The retainer ring 20 is moulded from synthetic plastic material and provided with a radially extending flange 22, and has a tapered upper portion 24 having a maximum diameter approximately equal to the internal diameter of the body 2 above the shelf 14, but greater than the internal diameter of the shelf, and a minimum diameter such that it can readily start to enter the tapered bottom entry to the body. A lower portion 26 of the ring above the flange 22 has a smaller external diameter and a height at least equal to the height of portion 16 so that the ring may be pressed into the tapered entry until a shoulder 28 between the upper and lower portions snaps over the shelf 14, thus positively retaining the ring. Preferably the lower portion 26 has an internal profile and height such as to provide snug accommodation of the portion 16 with due allowance for manufacturing tolerances. A small recess 30 may be formed in the inside wall of the body adjacent the shelf 14 to provide additional clearance for the shoulder 28.

The flange 22 has a limited radial extent so that it does not extend beyond a projection of the external surface of the vial body. The retainer ring (20) may thus be inserted prior to filling and capping, but it cannot of itself also provide a finger flange. This problem can be overcome if a flange is required by providing a separately moulded flange 32, with an annular forwardly projecting locking ring 34 which can be snapped into annular groove 36 formed within the ring 20 prior to use of the vial. Such a separable finger flange also has the advantage that it may be fitted if the syringe is to be operated manually, but omitted if the syringe is to be used in a syringe pump, since many syringe pumps are unable to accommodate projection beyond the general external cylindrical surface of a syringe body, which has tended to limit their use to plastic syringes designed for such use.

## Claims

1. A bottomless pharmaceutical vial for filling and capping prior to incorporation into a syringe, having a generally upright cylindrical hollow body (2) with a narrower filling neck (6) at its top end, a side wall of the body being formed with a bottom edge (8) surrounding a flared bottom opening, with an inner surface of a lower portion (16) of the side wall of the body adjacent said bottom opening extending upwardly to an outward joggle (12) in said inner surface, the joggle forming an upwardly facing annular shoulder (14), the lower portion of the side wall being substantially within an outside diameter of the cylindrical body; and a piston (10) within the body in sealing relation with an inner surface of the body above the outward jog; characterized by a piston retainer ring (20) inserted into the bottom opening of the body beneath the piston, the piston retainer ring having an outer surface with an upper portion tapered to enter the flare of the inner wall of the body adjacent said bottom opening, a lower portion of reduced external diameter, and a downwardly facing shoulder (28) connecting said upper and lower outer face portions, the ring being pressed into said bottom opening such that the downwardly facing shoulder snaps over the upwardly facing shoulder to retain the ring, no part of the retainer ring (20) extending radially outwardly of the external diameter of the cylindrical vial body, and the retainer ring incorporating means (36) by which a separately formed finger flange (32) extending radially outwardly of said outside diameter of the cylindrical body can be attached to the retainer ring following filling and capping of the vial.

2. A vial according to claim 1, characterized in that the retainer ring (20) includes a flange (22) extending beneath the bottom edge of the body but having a diameter no greater than that of the body.

3. A vial according to claim 2, characterized in that the flange (22) has an internal peripheral groove (36), and a separately formed finger flange (32) is provided with a locking ring for subsequent engagement with the groove.

4. A syringe assembly including a vial according to any of claims 1 to 3.

## Patentansprüche

1. Bodenloses pharmazeutisches Fläschchen zum Füllen und Deckeln vor dem Einbau in eine Spritze mit einem allgemein aufrechten zylindrischen, hohlen Körper (2) mit einem engeren Füllhals (6) an seinem oberen Ende, wobei eine Seitenwand des Körpers mit einer eine konisch erweiterte untere Öffnung umgebenden unteren Kante (8) ausgebildet ist, eine Innenfläche eines unteren Abschnitts (16) der Seitenwand des Körpers sich in der Nähe der unteren Öffnung nach oben zu einer äußeren Nase (12) an der Innenfläche erstreckt, wobei die Nase eine nach oben weisende ringförmige Schulter (14) bildet und der untere Abschnitt der Seitenwand im wesentlichen innerhalb eines Außendurchmessers des zylindrischen Körpers liegt, und einem Kolben (10) innerhalb des Körpers, der oberhalb der äußeren Nase dichtend an einer Innenfläche des Körpers anliegt, gekennzeichnet durch einen Kolbenhaltering (20), der in die untere Öffnung des Körpers unterhalb des Kolbens eingeführt ist und eine Außenfläche hat, die einen oberen Abschnitt, der zum Eintritt in die konische Erweiterung der Innenwand des Körpers in der Nähe der unteren Öffnung verjüngt ist, einen unteren Abschnitt mit kleinerem Außendurchmesser und eine nach unten weisende Schulter (28) aufweist, die den oberen und unteren Außenflächenabschnitt verbindet, wobei der Ring so in die untere Öffnung hineingepreßt wird, daß die nach unten weisende Schulter zur Halterung des Rings über die nach oben weisende Schulter einschnappt, wobei sich kein Teil des Halterings (20) radial über den Außendurchmesser des zylindrischen Fläschchenkörpers hinaus erstreckt und der Haltering eine Vorrichtung (36) beinhaltet, über die ein separat geformter Fingerflansch (32), der sich radial über den Außendurchmesser des zylindrischen Körpers hinaus erstreckt, nach dem Füllen und Deckeln des Fläschchens am Haltering angebracht werden kann.

2. Fläschchen nach Anspruch 1, dadurch gekennzeichnet, daß der Haltering (20) einen Flansch (22) aufweist, der sich zwar unterhalb der Unterkante des Körpers erstreckt, dessen Durchmesser jedoch nicht größer als der des Körpers ist.

3. Fläschchen nach Anspruch 2, dadurch gekennzeichnet, daß der Flansch (22) eine innere Umfangsnut (36) aufweist, und ein separat geformter Fingerflansch (32) mit einem Verriegelungsring versehen ist, der später in die Nut eingreift.

4. Spritzenanordnung mit einem Fläschchen nach einem der Ansprüche 1 bis 3.

## Revendications

1. Ampoule sans fond pour produits pharmaceutiques destinée à être remplie et fermée avant son incorporation dans une seringue, ayant un corps creux généralement cylindrique droit (2), avec un col (6) de remplissage plus étroit à son extrémité supérieure, une paroi latérale du corps étant formée avec un bord inférieur (8) entourant une ouverture de fond évasée, avec une surface interne d'une portion inférieure (16) de la paroi latérale du corps, adjacente à ladite ouverture de fond, s'étendant vers le haut jusqu'à un décrochement vers l'extérieur (12) dans ladite surface interne, le décrochement formant un épaulement annulaire (14) vers le haut, la portion inférieure de la paroi latérale étant substantiellement comprise dans un diamètre extérieur du corps cylindrique; et un piston (10) à l'intérieur du corps en relation de fermeture hermétique avec une surface interne du corps au-dessus du décrochement vers l'extérieur; caractérisée par un anneau (20) de retenue du piston inséré dans l'ouverture de fond du corps en dessous du piston, l'anneau de retenue du piston ayant une surface externe avec une portion supérieure amincie de manière à passer l'évasement de la paroi interne du corps adjacente à ladite ouverture de fond, une portion inférieure de diamètre extérieur réduit, et un épaulement vers le bas (28) connectant lesdites portions de face externe supérieure et inférieure, l'anneau étant pressé dans ladite ouverture de fond de telle sorte que l'épaulement vers le bas s'encliquète par-dessus l'épaulement vers le haut pour retenir l'anneau, aucune partie de l'anneau de retenue (20) ne s'étendant radialement vers l'extérieur du diamètre extérieur du corps d'ampoule cylindrique, et l'anneau de retenue incorporant un moyen (36) par lequel une bride pour les doigts (32) formée séparément, s'étendant radialement vers l'extérieur dudit diamètre extérieur du corps cylindrique peut être attachée à l'anneau de retenue suite au remplissage et à la fermeture de l'ampoule.

2. Ampoule selon la revendication 1, caractérisée en ce que l'anneau de retenue (20) comporte une bride (22) s'étendant en dessous du bord inférieur du corps, mais ayant un diamètre ne dépassant pas celui du corps.

3. Ampoule selon la revendication 2, caractérisée en ce que la bride (22) a une rainure périphérique interne (36), et une bride pour les doigts (32) formée séparément est pourvue d'un anneau de verrouillage pour l'engagement subséquent avec la rainure.

4. Ensemble de seringue comportant une ampoule selon l'une quelconque des revendications 1 à 3.
